# EUROPÄISCHE PATENTANMELDUNG

(11) **EP 4 806 409 A1**
(43) Veröffentlichungstag der Anmeldung: **16.09.2026**
(21) Anmeldenummer: 25209571.6
(22) Anmeldetag: 17.10.2025
(51) Int. Cl.: A61F 13/15, A44B 18/00, A61F 13/62, B29C 65/08, B29C 65/00

(54) **VERSCHLUSSSYSTEM MIT OPTIMIERTER OBERFLÄCHENQUALITÄT, HYGIENEARTIKEL MIT EINEM VERSCHLUSSSYSTEM UND VERFAHREN ZUM HERSTELLEN EINES VERSCHLUSSSYSTEMS**

(30) Priorität: 11.03.2025 DE 102025109236; 11.03.2025 DE 202025101295 U; 28.04.2025 DE 102025116333; 28.04.2025 EP 25173024
(71) Anmelder: Gottlieb Binder GmbH & Co. KG, 71088 Holzgerlingen (DE)
(72) Erfinder: HEEPE, Lars, 72072 Tübingen (DE); CARTER, Nick, 65191 Wiesbaden (DE)
(74) Vertreter: Kohler Schmid Möbus Patentanwälte

(57) **Zusammenfassung**

Die Erfindung betrifft ein Verschlusssystem (10), aufweisend ein Trägerteil (12) mit einer Rückseite (12') und einer Vorderseite (12"). Von der Vorderseite (12") stehen in einem regelmäßigem Muster Verschlusselemente (16, 16a, 16b, 16c) vor. Ein Festlegeteil (14) ist an der Rückseite (12') des Trägerteils (12) angeordnet. Das Trägerteil (12) und das Festlegeteil (14) sind durch mehrere Schweißstellen (18, 18a, 18b) bereichsweise miteinander verbunden. Neben einer jeweiligen Schweißstelle (18, 18a, 18b) definieren die freien Enden der Verschlusselemente (16, 16a, 16b, 16c) und eine vom Trägerteil (12) abgewandte Rückseite (14') des Festlegeteils (14) jeweils eine Referenzfläche (A, B), wobei ein jeweiliger Überstand (W) der Schweißstellen (18, 18a, 18b), welcher aus einem Bereich zwischen den Referenzflächen (A, B) hinausragt, höchstens 20 % einer zwischen den Referenzflächen (A, B) gemessenen Dicke (T) des Verschlusssystems (10) zu der jeweilig dem Überstand (W) nächstliegenden Referenzfläche (A, B) beträgt. Insbesondere stehen zumindest einige, vorzugsweise alle, der Schweißstellen (18, 18a, 18b) nicht aus dem Bereich zwischen den Referenzflächen (A, B) vor. Wenigstens eine der Schweißstellen (18, 18a, 18b), die von allen Rändern (24, 26) des Trägerteils (12) beabstandet ist, erfasst wenigstens ein ursprünglich am Trägerteil (12) vorhandenes Verschlusselement (16, 16a, 16b, 16c) und höchstens 10 ursprünglich am Trägerteil (12) vorhandene Verschlusselemente (16, 16a, 16b, 16c).

## Beschreibung

### Hintergrund der Erfindung

Die Erfindung betrifft ein Verschlusssystem, aufweisend ein Trägerteil von dessen Vorderseite in einem regelmäßigem Muster Verschlusselemente, insbesondere mit einer Höhe von höchstens 1 mm, bevorzugt höchstens 0,5 mm, vorstehen und weiterhin aufweisend ein Festlegeteil, das an der Rückseite des Trägerteils angeordnet ist. Das Trägerteil und das Festlegeteil sind durch mehrere Schweißstellen bereichsweise miteinander verbunden. Die Erfindung betrifft auch ein Verfahren zum Herstellen eines solchen Verschlusssystems und einen Hygieneartikel mit einem solchen Verschlusssystem.

Zum Herstellen eines Verschlusssystems, insbesondere eines Haftverschlusssystems, muss oftmals ein Trägerteil, welches in einem regelmäßigen Muster vorstehende Verschlusselemente aufweist, mit einem Festlegeteil verbunden werden. Das Festlegeteil kann dabei als Unterlagestruktur zur Abstützung bzw. Handhabung des Trägerteils bei der Verwendung des Verschlusssystems dienen. Es ist bekannt, das Trägerteil dazu mit dem Festlegeteil zu verschweißen, insbesondere mittels eines Ultraschallschweißverfahrens. Gegenüber der ebenfalls weit verbreiteten Methode des Verklebens hat dies den Vorteil, dass mittels eines rationellen Prozesses eine zuverlässige Verbindung zwischen Trägerteil und Festlegeteil, ggf. auch durch mehrere Lagen des Festlegeteils, erhalten werden kann. Insbesondere weist das resultierende Verschlusssystem eine größere Sortenreinheit auf, was sich unter anderem vorteilhaft bei der Entsorgung bzw. beim Recycling niederschlägt. Weiterhin müssen keine Überlegungen hinsichtlich der Haltbarkeit und der potentiellen Gesundheitsschädlichkeit eines Klebstoffes angestrengt werden.

Damit die Verbindung des Festlegeteils und des Trägerteils eine ausreichende Festigkeit aufweist, müssen erzeugte Schweißstellen eine gewisse Fläche einnehmen. Um dies zu ermöglichen, können Verschlusselemente des Trägerteils schlichtweg überschweißt werden. Aufgrund der zerstörten bzw. beschädigten Verschlusselemente an den entsprechenden Schweißstellen geht damit allerdings ein unwillkommener Verlust der potentiellen Haftkraft des Verschlusssystems einher. Darüber hinaus kann das Verschweißen ein Aufwölben bzw. Ausbeulen des Verschlusssystems an und nahe den Schweißstellen bedingen, was ebenfalls zu einer Störung beim Anhaften des Verschlusssystems an einem Gegenstück führen kann. Oftmals liegen derartige Aufwölbungen in einer größeren Größenordnung als die Abmessungen der Verschlusselemente eines Verschlusssystems. Entsprechende Aufwölbungen bzw. Beulen wirken sich daher insbesondere auch störend auf die Funktion von nicht zerstörten Verschlusselementen in unmittelbarer Nähe zu den Schweißstellen aus, da diesen durch die Aufwölbung eine abweichende Orientierung aufgeprägt wird und da ein vollflächiger Kontakt mit dem Gegenstück behindert wird. Insbesondere können dadurch auch optische und haptische Beeinträchtigungen des Verschlusssystems hervorgerufen werden.

EP 3 408 067 B1 beschreibt ein Verfahren zum Herstellen von Hygieneartikel-Verschlusssystemen, wobei ein Trägerteil, welches in Reihen angeordnete Verschlusselemente aufweist, mit einem Festlegeteil mittels eines Ultraschallschweißverfahrens verschweißt wird. Das Trägerteil wird dabei in einem kontinuierlichen Prozess entlang von Schweißverbindungslinien mit dem Festlegeteil verbunden. Die Schweißverbindungslinien werden zwischen benachbarten Reihen von Verschlusselementen eingebracht. Die Verschlusselemente werden durch den Schweißvorgang nicht beeinträchtigt.

EP 2 815 733 A1 offenbart ein ähnliches Verfahren zum Verschweißen eines Trägerteils mit einem Festlegeteil, wobei insbesondere ein Scheibenwerkzeug einer Ultraschallschweißanlage zur Erzeugung von Schweißverbindungslinien zwischen benachbarten Reihen von Verschlusselementen eingesetzt werden kann. Insbesondere können unterbrochene Schweißverbindungslinien erzeugt werden.

Bei den aus der EP 3 408 067 B1 bzw. EP 2 815 733 A1 bekannten Verfahren werden Verschlusselemente des jeweiligen Trägerteils während des Verschweißens zwar nicht beschädigt, allerdings müssen die Verfahren äußerst präzise durchgeführt werden, wobei die Geometrie der jeweiligen Ultraschallwerkzeuge auf die Anordnung der Verschlusselemente auf den jeweiligen Trägerteilen abgestimmt sein muss.

### Aufgabe der Erfindung

Es ist eine Aufgabe der Erfindung, ein zuverlässiges und bei Herstellung und Verwendung, insbesondere an einem Hygieneprodukt, vorteilhaftes Verschlusssystem anzugeben. Es ist auch Aufgabe der Erfindung, ein Verfahren zum Herstellen eines solchen Verschlusssystems sowie einen Hygieneartikel mit einem verbesserten Verschlusssystem bereitzustellen.

### Beschreibung der Erfindung

Diese Aufgabe wird erfindungsgemäß gelöst durch ein Verschlusssystem gemäß Patentanspruch 1, einen Hygieneartikel gemäß Patentanspruch 13 sowie ein Verfahren zum Herstellen eines Verschlusssystems gemäß Patentanspruch 14.

### Erfindungsgemäßes Verschlusssystem

Die Aufgabe wird mithin gelöst durch ein Verschlusssystem, aufweisend ein Trägerteil mit einer Rückseite und einer Vorderseite, von der in einem regelmäßigem Muster Verschlusselemente, insbesondere mit einer Höhe von höchstens 1 mm, bevorzugt höchstens 0,5 mm, vorstehen, und ein Festlegeteil, das an der Rückseite des Trägerteils angeordnet ist, wobei das Trägerteil und das Festlegeteil durch mehrere Schweißstellen bereichsweise miteinander verbunden sind, wobei neben einer jeweiligen Schweißstelle die freien Enden der Verschlusselemente und eine vom Trägerteil abgewandte Rückseite des Festlegeteils jeweils eine Referenzfläche definieren. Ein Überstand der Schweißstellen über die Referenzflächen beträgt höchstens 20 %, bevorzugt höchstens 10 %, besonders bevorzugt höchstens 5 %, einer zwischen den Referenzflächen gemessenen Dicke des Verschlusssystems. Insbesondere stehen die Schweißstellen nicht über die Referenzflächen hinaus. Wenigstens eine der Schweißstellen, die von Rändern des Trägerteils beabstandet ist, erfasst wenigstens ein ursprünglich am Trägerteil vorhandenes Verschlusselement. Zudem werden höchstens 10, insbesondere höchstens 5, vorzugsweise höchstens 3, ursprünglich am Trägerteil vorhandene Verschlusselemente von der erwähnten Schweißstelle erfasst. Diese (innenliegende) Schweißstelle, die ein oder wenige Verschlusselemente erfasst, ist grundsätzlich von allen Rändern des Trägerteils beabstandet. Insbesondere erfassen wenigstens 25 %, bevorzugt wenigstens 50 %, besonders bevorzugt wenigstens 75 %, der innenliegenden Schweißstellen zwischen 1 und 10 (bzw. 5 oder 3) ursprünglich vorhandene Verschlusselemente.

Vorzugsweise werden in einem ebenen Zustand des Verschlusssystems zwei gemeinsame Referenzflächen für alle Schweißstellen betrachtet. Die Referenzflächen werden insbesondere in einem Zustand definiert, in dem der Verbund aus Trägerteil und Festlegeteil eben ausliegt. Ggf. vorhandene Aufwölbungen oder Einprägungen an Schweißstellen bleiben dabei unberücksichtigt. Gleichermaßen werden bei der Definition der Referenzflächen ggf. vorhandene, materialbedingte oder handhabungsbedingte Aufwölbungen oder Knitterstellen am Verschlusssystem ignoriert.

Demnach wird bei der Bestimmung der Dicke des Verschlusssystems eine Referenzfläche von den Bereichen der Rückseite des Festlegeteils bestimmt, in denen keine Störungen der Oberfläche durch Schweißstellen vorhanden sind. Die Referenzfläche an der Rückseite des Festlegeteils ist insbesondere durch die am weitesten vom Trägerteil abgewandten Bereiche des Festlegteils definiert. Falls die Rückseite des Festlegeteils einzelne vorstehende Fasern aufweist, können diese ignoriert werden, beispielsweise indem die Referenzfläche so gewählt ist, dass 98 % der Masse des Festlegeteils auf der trägerteilseitigen Seite der Referenzfläche liegen.

Die andere Referenzfläche wird von den am weitesten von der Vorderseite des Trägerteils entfernten freien Enden der Verschlusselemente des Trägerteils bestimmt, welche nicht durch eine Verschweißung beeinflusst wurden. Sollten Verschlusselemente eines Trägerteiles unterschiedliche Höhen aufweisen, wird die Referenzfläche vorzugsweise von den am weitesten vorstehenden Verschlusselementen bestimmt.

Die Referenzflächen bilden typischerweise zwei zueinander parallele Ebenen.

Die Höhe eines jeweiligen Verschlusselements ist über den Abstand seines freien Endes zu der Vorderseite des Trägerteils bestimmt. Dabei wird orthogonal vom freien Ende auf die Vorderseite des Trägerteils gemessen.

Als ursprünglich am Trägerteil vorhandene Verschlusselemente werden jene Verschlusselemente bezeichnet, welche vor dem Verschweißen des Trägerteils mit dem Festlegeteil (aufgrund der regelmäßigen Anordnung) vorhanden waren und welche beim Verschweißen ganz oder teilweise überschweißt wurden.

Unter dem Begriff der "Schweißstelle" bzw. der "Verschweißung" wird typischerweise eine stoffschlüssige Verbindung zumindest zweier Werkstücke (hier Trägerteil und Festlegeteil) verstanden, welche unter Aufschmelzen oder Plastifizieren der Werkstücke zustande gekommen ist. Im vorliegenden Zusammenhang kann darunter weiterhin ein auf einem Formschlussprinzip basierender Verbindungsmechanismus verstanden werden, welcher eine makroskopische Vermischung von Bestandteilen der Werkstücke vorsieht. Beispielsweise kann dazu eines der Werkstücke, insbesondere das Trägerteil, bereichsweise aufgeschmolzen oder plastifiziert worden sein und in eine poröse Struktur des anderen Werkstücks, insbesondere des Festlegeteils, eingebracht worden sein. Nach dem Erstarren des aufgeschmolzenen/plastifizierten Materials besteht eine formschlüssige Verbindung zwischen den Werkstücken. Insbesondere kann an zumindest einer Schweißstelle eine Kombination der beiden genannten Verbindungsprinzipien vorliegen.

Bevorzugt weisen das Trägerteil und/oder das Festlegeteil thermoplastisches Kunststoffmaterial, insbesondere Polyolefine, Polyester und/oder Polyamid, auf. Weiter bevorzugt weisen das Trägerteil und/oder das Festlegeteil biologisch abbaubares Kunststoffmaterial und/oder biobasiertes Kunststoffmaterial auf. Weisen beide Teile die erwähnten thermoplastischen (und somit schweißbaren) Kunststoffmaterialien auf, können Schweißstellen insbesondere nach dem Stoffschlussprinzip ausgebildet sein. Weiter bevorzugt kann das Verschlusssystem, insbesondere das Festlegeteil, organisches Fasermaterial, insbesondere natürliches Fasermaterial, besonders bevorzugt Baumwoll- oder Hanffasern, aufweisen. Dies kann die Ausbildung von Schweißstellen nach dem Formschlussprinzip unterstützen.

Insbesondere können zumindest einige Schweißstellen vorhanden sein, welche keine der ursprünglich am Trägerteil vorhandenen Verschlusselemente erfassen. Typischerweise erfasst die Mehrzahl der vorhandenen Schweißstellen allerdings zumindest eines der ursprünglich vorhandenen Verschlusselemente.

Vorzugsweise erfassen alle der an dem Verschlusssystem vorhandenen, voneinander beabstandeten Schweißstellen jeweils höchstens 10, insbesondere höchstens 5, vorzugsweise höchstens 3, der ursprünglich am Trägerteil vorhandenen Verschlusselemente. Neben einer Begrenzung des durch überschweißte Verschlusselemente bedingten lokalen Haftfähigkeitsverlustes ist dadurch sichergestellt, dass der Verbund aus Trägerteil und Festlegeteil keine übermäßigen, insbesondere richtungsabhängigen, Steifigkeitswerte aufweist. Insbesondere kann durch das Festlegen der Positionen der Schweißstellen im Rahmen der Auslegung des Verschlusssystems die Steifigkeitsverteilung des Verschlusssystems und damit auch dessen Ansprechverhalten bei Betätigung in vorteilhafter Weise beeinflusst werden.

Das erfindungsgemäße Verschlusssystem zeichnet sich durch einen zuverlässigen Halt des Trägerteils am Festlegeteil aus, wobei die Haftfähigkeit des Verschlusssystems trotz der überschweißten Verschlusselemente nur unwesentlich gemindert wird. Indem ein Verschlusselement oder wenige Verschlusselemente pro Schweißstelle überschweißt werden können, wird die Herstellung vereinfacht. Es braucht insbesondere nicht auf die präzise Positionierung der Schweißstellen in Zwischenräumen zwischen Verschlusselementen geachtet zu werden. Ferner wird die Haftfähigkeit nicht durch übermäßige Aufwölbungen des Verbundes aus Trägerteil und Festlegeteil aus der Hauptebene des Verschlusssystems heraus und den damit einhergehenden Orientierungsänderungen der benachbarten Verschlusselemente und/oder ein nur lokal begrenztes Ineinandergreifen von Verschlusselementen und Gegenstück gemindert. Insbesondere ist durch die Vermeidung von Aufwölbungen ein vorteilhaftes Erscheinungsbild und eine vorteilhafte Haptik des Verschlusssystems bedingt. Idealerweise sind individuelle Schweißstellen von einem Endnutzer nicht unmittelbar fühlbar.

In Ausführungsformen sind die Schweißstellen allesamt gleichartig ausgebildet und/oder in einem regelmäßigen Muster angeordnet.

Das Muster der Anordnung der Schweißstellen unterscheidet sich dabei im Allgemeinen vom Muster der Anordnung der Verschlusselemente.

Die gleichartige Ausbildung der Schweißstellen umfasst insbesondere deren jeweilige flächige Ausdehnung. Die genauen Abmessungen können dabei vorzugsweise über ein zum Verschweißen verwendetes Werkzeug vorgegeben werden. Die jeweilige detaillierte Ausbildung verschiedener Schweißstellen, insbesondere im Hinblick auf die Position und/oder Anzahl der überschweißten Verschlusselemente innerhalb der jeweiligen Schweißstelle, kann gewissen Schwankungen unterliegen.

Über die gleichartige Ausbildung bzw. regelmäßige Anordnung der Schweißstellen kann eine einfache Auslegung und Durchführung eines Herstellungsprozesses des Verschlusssystems erreicht werden. Insbesondere können die Herstellung und der Einsatz der benötigten Werkzeuge vereinfacht werden.

In alternativen Ausführungsformen können insbesondere die Schweißstellen an den Rändern des Trägerteils eine andere Gestaltung aufweisen als die innenliegenden Schweißstellen.

Unter den Rändern des Trägerteils werden in diesem Zusammenhang insbesondere dessen Längsränder verstanden, an welchen das Trägerteil und das Festlegeteil des Verschlusssystems typischerweise nicht gemeinsam abschließen. Insbesondere erstreckt sich das Festlegeteil dabei über den jeweiligen Längsrand des Trägerteils hinaus. Veranschaulicht werden kann dies, indem man sich einen Herstellungsprozess vorstellt, wobei ein bandförmiges Trägerteil mit einem bandförmigen Festlegeteil entlang einer Maschinenrichtung zusammengeführt und verschweißt wird und wobei im Nachgang mehrere Verschlusssysteme durch Zuschneiden des bandförmigen Verschlusssystems quer zur Maschinenrichtung erhalten werden. An den Schnittkanten schließen das Trägerteil und das Festlegeteil dabei gemeinsam ab. Diese Schnittkanten werden im vorliegenden Zusammenhang als in Querrichtung verlaufende Querränder bezeichnet. Die Längsränder des Trägerteils verlaufen insbesondere senkrecht zu den Querrändern, typischerweise parallel zu der Maschinenrichtung. Im Falle, dass die Längsränder und/oder Querränder des Trägerteils nicht als senkrecht zueinanderstehende Geraden ausgebildet sind (beispielsweise könnten Ränder eine Wellenform aufweisen) gilt obenstehendes sinngemäß für den gemittelten Verlauf der Längsränder und der Querränder.

Schweißstellen an den Rändern, insbesondere Längsrändern, des Trägerteils können zur Fixierung des jeweiligen Randes diesen überlappen. Mit anderen Worten kann eine jeweilige Schweißstelle einen der Ränder des Trägerteils derart überlappen, dass ein über den Rand hinausragender Bereich des Festlegeteils Teil der jeweiligen Schweißstelle ist. Dabei gibt es am über den Rand hinausreichenden Teil der entsprechenden Schweißstelle keine Verbindung zwischen Trägerteil und Festlegeteil, sondern lediglich eine Schweißung innerhalb des Festlegeteils, sofern das Festlegeteil einen schweißbaren Materialbestandteil aufweist. Lediglich am im Bereich des Randes befindlichen Teil der jeweiligen Schweißstelle verbindet diese das Trägerteil mit dem Festlegeteil. Falls das Festlegeteil keinen schweißbaren Materialbestandteil oder insbesondere große Anteile nichtschweißbaren Materials aufweist (d. h. an Schweißstellen kommt vornehmlich das oben beschriebene Formschlussprinzip zum Einsatz), kann vorzugsweise schweißbares Material des Trägerteils beim Verschweißen bis über den Rand hinaus in die Materialstruktur des Festlegeteils eingeflossen sein. Derart kann sichergestellt werden, dass der Rand des Trägerteils an der entsprechenden Schweißstelle zuverlässig mit dem Festlegeteil verbunden ist.

Die beschriebene Fixierung der Ränder des Trägerteils kann insbesondere Vorteile bezüglich der Haptik und/oder der Scherstandfestigkeit des Verschlusssystems bergen.

Um keine übermäßigen Anforderungen an die Präzision eines entsprechenden Herstellungsverfahrens stellen zu müssen und die Fertigungsgeschwindigkeit erhöhen zu können, kann dennoch bevorzugt werden den Rändern des Trägerteils keine gesonderte Behandlung zukommen zu lassen, sondern eine einfache regelmäßige Verteilung der Schweißstellen, insbesondere unabhängig von deren potentiellem Zusammenfallen mit den Rändern des Trägerteils, vorzusehen. Dies wird begünstigt, indem viele kleine Schweißstellen in geringen Abständen voneinander vorgesehen sind.

Vorzugsweise erstrecken sich zumindest einige der Schweißstellen, insbesondere diejenigen, welche ursprünglich am Trägerteil vorhandene Verschlusselemente erfassen, insbesondere alle Schweißstellen geradlinig. Weiter bevorzugt sind die geradlinigen Schweißstellen parallel zueinander ausgerichtet.

In einer, typischerweise der erwähnten Maschinenrichtung entsprechenden, Längsrichtung des Trägerteils ergeben sich dabei gerade Schweißstellen, deren Ausdehnung in Längsrichtung zumindest geringfügig größer ist als deren Ausdehnung in Querrichtung.

Die Verteilung der geradlinigen Schweißstellen in der Ebene des Trägerteils kann zur Ausbildung des erwähnten Musters insbesondere regelmäßig sein. Im Rahmen der Auslegung des Verschlusssystems kann, wie erwähnt, insbesondere über die Verteilungsdichte der Schweißstellen und/oder deren vorgesehene Tiefe die Steifigkeitsverteilung des Verschlusssystems aktiv beeinflusst werden.

Die Verschlusselemente sind vorzugsweise in einem hexagonalen Muster und/oder in schräg zu der Längsrichtung des Trägerteils, insbesondere schräg zu den geradlinigen Schweißstellen, verlaufenden Reihen angeordnet.

Ein entsprechendes Muster der Verschlusselemente ergibt sich beispielsweise, wenn diese in Querreihen am Trägerteil angeordnet sind, wobei die Verschlusselemente unmittelbar benachbarter Querreihen jeweils versetzt zueinander angeordnet sind, insbesondere wobei die Distanz der Verschlusselemente zu ihren jeweilig unmittelbar benachbarten Verschlusselementen jeweils gleich ist. Entlang der Längsrichtung des Trägerteils geradlinige Schweißstellen verlaufen dann senkrecht zu den Querreihen der Verschlusselemente und schräg zu sekundären Reihen der Verschlusselemente.

Die beschriebene Anordnung der Verschlusselemente auf dem Trägerteil entspricht einer bekannten und bewährten Anordnung von Verschlusselementen bekannter Verschlusssysteme. Bei dieser Anordnung ist es jedoch mit rationellen Fertigungsmethoden kaum möglich alle Schweißstellen zwischen den Verschlusselementen einzubringen. Das erfindungsgemäße Überschweißen weniger Verschlusselemente pro Schweißstelle ermöglicht es, hohe Fertigungsgeschwindigkeiten zu erreichen. Gleichzeitig wird durch die flache Ausbildung der Schweißstellen die Haptik und Gebrauchstauglichkeit, insbesondere Festigkeit der Verbindung mit einem Gegenstück allenfalls geringfügig oder vorteilhafterweise nicht beeinträchtigt.

Bevorzugt sind höchstens 20 %, insbesondere höchstens 15 %, der ursprünglich vorhandenen Verschlusselemente von Schweißstellen erfasst.

Dies gilt ggf. unter Betrachtung des gesamten Verschlusssystems in den Abmessungen, in denen es nach der Herstellung zum Einsatz kommt, beispielsweise in Form eines Windelverschlusses. Insbesondere gilt dies bei Betrachtung eines Abschnitts des Verschlusssystems, der in Längsrichtung des Trägerteils länger als 1 cm ist.

Werden nicht mehr als 20 % der Verschlusselemente beim Verschweißen des Trägerteils mit dem Festlegeteil beeinträchtigt, ergibt sich insbesondere eine hohe Haftfähigkeit des Verschlusssystems.

Bevorzugt entspricht eine Breite der Schweißstellen höchstens dem Abstand zweier benachbarter Verschlusselemente und/oder höchstens einer Querausdehnung eines Verschlusselements, insbesondere eines überschweißten Verschlusselements.

Die Breite einer Schweißstelle wird insbesondere quer zu einer Längserstreckung der Schweißstelle, typischerweise in Querrichtung des Trägerteils, gemessen. Die Querausdehnung eines Verschlusselementes bezeichnet dessen maximale Ausdehnung, insbesondere die maximale Ausdehnung im Bereich des freien Endes des Verschlusselementes, in Querrichtung des Trägerteils.

Bei einer derartigen Ausbildung der Schweißstellen kann eine jeweilige Schweißstelle ggf. in einem Zwischenraum zwischen zwei in Querrichtung unmittelbar benachbarten Verschlusselementen liegen. Darüber hinaus wird ggf. nur ein ursprünglich vorhandenes Verschlusselement einer Querreihe von Verschlusselementen durch eine jeweilige Schweißstelle erfasst.

In der Folge bleibt eine lokale Haftfähigkeit des Verschlusssystems in Querrichtung des Trägerteils durch verbleibende umliegende Verschlusselemente erhalten. Insbesondere ergibt sich eine geringe Rauigkeit des Verschlusssystems und schließlich eine angenehme Haptik.

Typischerweise beträgt der Abstand zwischen unmittelbar benachbarten Verschlusselementen des Verschlusssystems weniger als 1 mm. Die maximale Querausdehnung der Verschlusselemente liegt vorzugsweise unter 1 mm, besonders bevorzugt unter 0,5 mm.

Weiter bevorzugt weisen zumindest einige, insbesondere alle, Schweißstellen in der Längsrichtung des Trägerteils eine Längserstreckung auf, die wenigstens dem Abstand zweier in der Längsrichtung unmittelbar aufeinanderfolgender Verschlusselemente und/oder höchstens dem Abstand dreier in der Längsrichtung unmittelbar aufeinanderfolgender Verschlusselemente entspricht.

Besonders bevorzugt sind an zumindest einigen der Schweißstellen beide der vorgenannten Kriterien zur Breite und Längserstreckung realisiert. Durch eine Längserstreckung einer jeweiligen Schweißstelle, welche größer als der Abstand zweier Verschlusselemente ist, kann eine ausreichende Festigkeit der Verbindung zwischen Trägerteil gewährleistet sein, während durch die Begrenzung der Längserstreckung sowie der Breite wiederum die lokale Haftfähigkeit und die geringe Rauigkeit des Verschlusssystems erhalten wird.

Vorzugsweise folgen die von einer jeweiligen Schweißstelle erfassten ursprünglich vorhandenen Verschlusselemente entlang einer geraden Linie aufeinander.

Wenn eine jeweilige Schweißstelle des erfindungsgemäßen Verschlusssystems lediglich ursprünglich vorhandene Verschlusselemente erfasst, welche auf einer geraden Linie aufeinanderfolgen bzw. aufeinanderfolgten, kann eine mehrdimensionale Beeinträchtigung der Verschlusselemente im Umfeld der Schweißstelle vermieden werden. Zur Veranschaulichung kann beispielsweise ein kartesisches Koordinatensystem an das Verschlusssystem angelegt werden, wobei die X-Achse der Längsrichtung des Trägerteils und die Y-Achse der Querrichtung des Trägerteils entspricht. Dann werden durch eine mehrere Verschlusselemente erfassende Schweißstelle mit einer Längserstreckung entlang der X-Achse und insbesondere einer maximalen Breite entsprechend der Querausdehnung eines Verschlusselementes lediglich entlang der Richtung der X-Achse mehrere aufeinanderfolgende Verschlusselemente beeinträchtigt. Die Ausbildung von flächig ausgedehnten Bereichen verminderter Haftfähigkeit wird vermieden.

Bei makroskopischen, durch Verschweißungen hervorgerufenen, Aufwölbungen in Richtung der Z-Achse eines entsprechenden kartesischen Koordinatensystems, welche sich vielfach an bekannten Verschlusssystemen finden, ist dies demgegenüber nicht gegeben. Vielmehr wird die lokale Haftfähigkeit des Verschlusssystems in diesen Fällen sowohl entlang der X-Achse als auch entlang der Y-Achse wesentlich beeinträchtigt.

Vorzugsweise sind die Schweißstellen in Längsreihen angeordnet, wobei Schweißstellen unmittelbar benachbarter Längsreihen einander bei einer Betrachtung quer zu den Längsreihen in der Längsrichtung überlappen.

Dadurch kann insbesondere eine gleichmäßige Steifigkeitsverteilung und damit eine über die gesamte Ausdehnung des Verschlusssystems einheitliche Handhabung sowie Haptik desselben gegeben sein.

Insbesondere sind zumindest drei derartige Längsreihen an dem Trägerteil angeordnet. Bevorzugt sind zumindest fünf Längsreihen der Schweißstellen an dem Trägerteil angeordnet.

Vorzugsweise kann das Verschlusssystem ein Gegenstück zum Zusammenwirken mit den Verschlusselementen aufweisen. Insbesondere ist durch das Überschweißen der ursprünglich vorhandenen Verschlusselemente eine Festigkeit der Verbindung des Trägerteils des Verschlusssystems mit dem Gegenstück um höchstens 20 % gegenüber einer Verbindung des Gegenstücks mit einem gleichartigen Trägerteil, bei dem alle Verschlusselemente intakt sind (welches insbesondere nicht mit einem Festlegeteil verschweißt ist), verringert.

Das Gegenstück kann insbesondere ein Vlies, ein Gewebe oder ein Gestrick aufweisen, welches sich zum Verhaken mit den Verschlusselementen des Trägerteils eignet. Insbesondere kann das Gegenstück ein Abschnitt einer Landing Zone einer Windel sein.

Zur Beurteilung der Festigkeit der Verbindung zwischen dem Trägerteil und dem Gegenstück kann vorzugsweise die Scherstandfestigkeit des Verschlusssystems herangezogen werden. Diese lässt sich insbesondere experimentell ermitteln. Beispielsweise kann nach einem kontrollierten (anpressdruckgesteuerten) Verschließen eines Verschlusssystems mit einer vorgegebenen Verschlussfläche (Trägerteilgrundfläche) das Verschlusssystem mit einer vorgegebenen Masse unter kontrollierten atmosphärischen Bedingungen in einer bestimmten Richtung auf Scherung belastet werden, wobei die Dauer der Verschlussintegrität gemessen wird.

Es besteht nicht notwendigerweise ein linearer Zusammenhang zwischen dem Anteil der von den Schweißstellen erfassten ursprünglich vorhandenen Verschlusselemente und dem Abfallen der Scherstandfestigkeit.

Vorzugsweise weisen die Verschlusselemente des Verschlusssystems an den freien Enden Köpfe auf, die zumindest in einer Richtung, insbesondere allseitig, über einen Stiel des jeweiligen Verschlusselements vorstehen.

Insbesondere stehen die Köpfe in einer sich radial von der Hochachse des Stiels erstreckenden Richtung über den Stiel vor.

Insbesondere können die Verschlusselemente hakenförmig, pilzförmig, palmenförmig oder hyperboloidförmig ausgebildet sein.

Vorzugsweise weist das Verschlusssystem wenigstens ein weiteres Trägerteil auf, das wie das erste Trägerteil mit dem Festlegteil verbunden ist. Insbesondere können sich die mehreren Trägerteile parallel zueinander erstrecken.

Zwei Trägerteile sind insbesondere in der Querrichtung zueinander beabstandet auf dem Festlegeteil angeordnet. Ggf. können mehr als zwei Trägerteile auf einem Festlegeteil angeordnet sein. Bevorzugt befinden sich zwei bis zehn Trägerteile, besonders bevorzugt zwei bis sieben Trägerteile, ganz besonders bevorzugt zwei bis fünf Trägerteile auf einem Festlegeteil. Sind mehr als zwei Trägerteile auf einem Festlegeteil angeordnet, können diese in der Querrichtung zu ihren jeweiligen unmittelbar benachbarten Trägerteilen wahlweise jeweils gleiche oder unterschiedliche Abstände aufweisen. Abstände zwischen jeweiligen unmittelbar benachbarten Trägerteilen liegen insbesondere im Bereich von 0,3 mm bis 30 mm, besonders bevorzugt von 0,5 mm bis 15 mm, ganz besonders bevorzugt von 1 mm bis 5 mm.

Insbesondere können Trägerteile eines Festlegeteils unterschiedliche Ausdehnungen in der Querrichtung aufweisen. Bevorzugt weisen Trägerteile Querausdehnungen von 1 mm bis 30 mm, besonders bevorzugt von 1,5 mm bis 15 mm, ganz besonders bevorzugt von 2 mm bis 10 mm, auf.

Trägerteile eines Festlegeteils können sich insbesondere hinsichtlich der Ausbildung der Verschlusselemente und/oder des Anordnungsmusters ihrer Verschlusselemente unterscheiden. Gleichermaßen kann sich das Anordnungsmuster der Schweißstellen verschiedener Trägerteile und oder deren jeweilige Ausdehnungen und/oder deren Anzahl auf verschiedenen Trägerteilen unterscheiden.

Beispielsweise kann ein Festlegeteil fünf Trägerteile aufweisen, die sich entlang der Längsrichtung der Trägerteile über das Festlegeteil erstrecken, wobei zwei unterschiedliche Typen von Trägerteilen vorhanden sind. Beispielsweise kann ein Trägerteiltyp pilzförmige Verschlusselemente aufweisen, während der andere Trägerteiltyp hyperboloidförmige Verschlusselemente aufweisen kann. Alternativ oder zusätzlich kann ein Trägerteiltyp dichter angeordnete Verschlusselemente aufweisen als der andere Trägerteiltyp.

Unabhängig vom jeweiligen Trägerteiltyp können Trägerteile eines Festlegeteils verschiedene Ausdehnungen in der Querrichtung aufweisen.

Die Anordnung von Trägerteilen eines bestimmten Typs und oder einer bestimmten Ausdehnung kann symmetrisch oder asymmetrisch zu einer Mittelachse der gesamten Trägerteilanordnung des Verschlusssystems erfolgen.

Denkbar ist, dass zur Fixierung der Trägerteile Schweißstellen in einem regelmäßigen Muster über die gesamte Trägerteilanordnung des Verschlusssystems verteilt sind, insbesondere wobei keine präzise Abstimmung des Schweißstellenmusters zur Zusammensetzung der Trägerteilanordnung vorgenommen ist. Insbesondere erfasst das Schweißstellenmuster dabei auch Bereiche des Festlegeteils, welche zwischen den in Querrichtung beabstandeten Trägerteilen liegen. Dabei werden insbesondere Einprägungen im Festlegeteil erzeugt.

Alternativ können spezielle Schweißmuster und/oder speziell geformte Schweißstellen an den jeweiligen Rändern der Trägerteile angeordnet sein, um diese in zuverlässiger Weise zu fixieren. Ggf. kann diese besondere Fixierung nur an den jeweiligen äußersten Längsrändern der Trägerteilanordnung realisiert sein (d. h. nur an jeweils einem äußeren Längsrand der äußeren Trägerteile, nicht aber am inneren Längsrand der äußeren Trägerteile und auch nicht an Längsrändern der inneren Trägerteile, soweit vorhanden).

### Erfindungsgemäßer Hygieneartikel

Die Aufgabe wird auch gelöst durch einen Hygieneartikel, insbesondere eine Windel, mit einem zuvor beschriebenen Verschlusssystem. Insbesondere weist der Hygieneartikel ein Verschlusssystem auf, bei dem ein Festlegeteil mehrere Trägerteile aufweist.

Das Festlegeteil kann in Ausführungsformen ein Windelohr einer Windel sein. Alternativ kann das Festlegeteil eine Lasche darstellen, welche wiederum an ein Windelohr angebunden sein kann. Typischerweise ist das Windelohr an einem Hauptkörper der Windel befestigt oder ausgebildet. Insbesondere kann das Windelohr monolithisch mit dem Hauptkörper ausgebildet sein. Die Windel weist insbesondere zwei Festlegeteile und zumindest ein Gegenstück zum Verhaken mit den Festlegeteilen auf. Typischerweise wird das Gegenstück durch eine äußere Schicht des Hauptkörpers im Frontbereich der Windel (Landing Zone) ausgebildet.

Der Hygieneartikel lässt sich einfach produzieren. Das erfindungsgemäße Verschlusssystem kann den Komfort bei der Verwendung des Hygieneartikels erhöhen. Zugleich wird das Trägerteil zuverlässig am Festlegeteil fixiert. Da bei einem Verschweißen von Trägerteil und Festlegeteil keine zusätzliche Rohstoffkomponente in den Verbund eingebracht wird, gestaltet sich das Recycling des Hygieneartikels vergleichsweise unproblematisch.

### Erfindungsgemäßes Verfahren

Die Aufgabe wird ferner gelöst durch ein Verfahren zum Herstellen eines zuvor beschriebenen Verschlusssystems, wobei zum Verschweißen ein Trägerteil und ein Festlegteil zwischen einem, vorzugsweise rotierenden, Amboss und einer Sonotrode einer Ultraschallschweißanlage durchgeführt werden. Mit diesem Verfahren kann das erfindungsgemäße Verschlusssystem in rationeller und zuverlässiger Weise hergestellt werden.

Der Amboss kann insbesondere walzenförmig ausgebildet sein. Wahlweise kann er frei drehbar angeordnet sein oder durch eine Antriebseinheit angetrieben werden. Die Sonotrode kann insbesondere flach ausgebildet sein. Bevorzugt ist die Sonotrode jedoch ebenfalls walzenförmig ausgebildet. Die walzenförmige Sonotrode ist typischerweise um eine zur Drehachse des Ambosses parallele weitere Drehachse drehbar gelagert.

Schweißstellen werden vorzugsweise zumindest teilweise durch Verschmelzen von Materialbestandteilen des Trägerteils und von Materialbestandteilen des Festlegeteils (Stoffschlussprinzip) und/oder zumindest teilweise durch Umschließen von nicht aufgeschmolzenen Materialbestandteilen des einen Teils, insbesondere des Festlegeteils, durch Materialbestandteile des anderen Teils, insbesondere des Trägerteils erzeugt (Formschlussprinzip).

Die Ausbildung und Positionierung der Schweißstellen erfolgt insbesondere nach den zum erfindungsgemäßen Verschlusssystem erläuterten Prinzipien.

Während des Verschweißens kann das Festlegeteil insbesondere gemeinsam mit dem Trägerteil relativ zu der Ultraschallschweißanlage in der Maschinenrichtung verschoben werden. Die Maschinenrichtung entspricht dabei, wie erwähnt, typischerweise der Längsrichtung des Trägerteils. Gleichermaßen kann die Ultraschallschweißanlage selbst entlang der Maschinenrichtung verschoben werden. Die zu verbindenden Teile können dabei unbewegt gehalten werden oder ebenfalls verschoben werden.

Vorzugsweise wird das erfindungsgemäße Verfahren in einem kontinuierlichen Prozess durchgeführt, wobei zumindest eine Bahn von Trägerteilmaterial mit zumindest einer Bahn von Festlegeteilmaterial zusammengeführt wird und diese Bahnen miteinander verschweißt werden. Ggf. kann im Anschluss das Verschlusssystem bzw. können mehrere Verschlusssysteme von der resultierenden Verbundbahn abgetrennt werden.

Vorzugsweise beträgt eine Vorschubgeschwindigkeit entlang der Maschinenrichtung wenigstens 70 m/min, vorzugsweise wenigstens 100 m/min, weiter bevorzugt wenigstens 150 m/min, besonders bevorzugt wenigstens 300 m/min, ganz besonders bevorzugt wenigstens 500 m/min. Die Vorschubgeschwindigkeit bezeichnet die Geschwindigkeit mit der sich die Trägerteilbahn bzw. das Trägerteil und die Festlegeteilbahn bzw. das Festlegeteil beim Verbinden relativ zu der Ultraschallschweißanlage bewegen. Falls der Vorschub nicht gleichmäßig erfolgt, bezeichnet die Vorschubgeschwindigkeit eine mittlere Vorschubgeschwindigkeit. Die Mittelung erfolgt dabei insbesondere über die zur Erzeugung eines Verschlusssystems benötigte Zeitspanne.

Bei einer bevorzugten Variante des Verfahrens wird zugleich eine Mehrzahl von Trägerteilen bzw. Trägerteilbahnen mit wenigstens einem Festlegeteil bzw. wenigstens einer Festlegeteilbahn verschweißt. Gleichermaßen können insbesondere jeweils ein (einziges) Trägerteil oder mehrere Trägerteile mit mehreren Festlegeteilen im Rahmen einer Parallelbearbeitung an einer Ultraschallschweißanlage bearbeitet werden. Werden zeitgleich mehrere Trägerteile verarbeitet, kann es sich dabei insbesondere um verschiedenartige Trägerteile handeln. D. h. die Ausbildung und/oder Anordnung der Verschlusselemente der Trägerteile und/oder die Ausdehnung der jeweiligen Trägerteile kann ggf. jeweils unterschiedlich sein.

Vorzugsweise liegt der Amboss beim Verschweißen am Trägerteil und die Sonotrode am Festlegeteil an.

Der rotierende Amboss kann insbesondere am Trägerteil abrollen. Falls die Sonotrode flach ausgebildet ist, gleitet das Festlegeteil während des Verschweißens insbesondere an ihr entlang.

Der Amboss weist vorzugsweise mehrere, insbesondere in Umfangsrichtung voneinander beabstandete, Schweißvorsprünge auf.

Die Schweißvorsprünge dienen im Zusammenwirken mit der Sonotrode zur Erzeugung der diskreten Schweißstellen in den zum erfindungsgemäßen Verschlusssystem beschriebenen Ausbildungen. Demnach liegt der Amboss beim Verschweißen mit den Schweißvorsprüngen am Trägerteil an.

Die Schweißvorsprünge können vorzugsweise jeweils identisch ausgeformt sein und/oder in einem regelmäßigen Muster am Umfang des Ambosses angeordnet sein. Alternativ können Schweißvorsprünge unterschiedliche Ausformungen aufweisen. Beispielsweise können zumindest einige der Schweißvorsprünge jeweils unterschiedliche Höhen (in radialer Richtung des Ambosses) aufweisen, wodurch ggf. Schweißstellen mit unterschiedlichen Eigenschaften erhalten werden können. Alternativ oder zusätzlich kann es sinnvoll sein, speziell ausgeformte Schweißvorsprünge zur Fixierung eines Randes eines Trägerteils vorzusehen.

Eine Wirkoberfläche der Sonotrode kann glatt ausgebildet sein.

Verschlusselemente, welche beim Verschweißen nicht von Schweißvorsprüngen erfasst werden, werden beim Durchführen zwischen Sonotrode und Amboss vom Amboss vorzugsweise nicht beeinträchtigt, insbesondere nicht vom Amboss berührt.

Mittels des erfindungsgemäßen Verfahrens lässt sich das erfindungsgemäße Verschlusssystem in rationeller Weise herstellen.

Weitere Merkmale und Vorteile der Erfindung ergeben sich aus der Beschreibung, den Ansprüchen und der Zeichnung. Erfindungsgemäß können die vorstehend genannten und die noch weiter ausgeführten Merkmale jeweils einzeln für sich oder zu mehreren in beliebigen, zweckmäßigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Kurzbeschreibung der Zeichnung

Die Erfindung ist in der Zeichnung dargestellt und wird anhand von Ausführungsbeispielen beschrieben. Es zeigen:
- Fig. 1: eine perspektivische Ansicht eines bekannten Verschlusssystems;
- Fig. 2: eine schematische Seitenansicht eines bekannten Verschlusssystems;
- Fig. 3: eine schematische Draufsicht auf ein erfindungsgemäßes Verschlusssystem;
- Fig. 4: eine schematische Seitenansicht eines erfindungsgemäßen Verschlusssystems;
- Fig. 5: eine schematische Seitenansicht eines weiteren erfindungsgemäßen Verschlusssystems;
- Fig. 6: eine schematische Draufsicht auf ein weiteres erfindungsgemäßes Verschlusssystem;
- Fig. 7: einen erfindungsgemäßen Hygieneartikel, in einer schematischen Perspektivansicht;
- Fig. 8: einen weiteren erfindungsgemäßen Hygieneartikel, in einer schematischen Perspektivansicht;
- Fig. 9: eine Ultraschallschweißanlage bei der Durchführung eines erfindungsgemäßen Verfahrens.

### Detaillierte Beschreibung der Erfindung und Zeichnung

In den Figuren der Zeichnung sind mehrfach auftretende Elemente zur besseren Übersichtlichkeit i. d. R. nur zum Teil mit Bezugszeichen versehen. Alphanumerische Bezugszeichen werden im Kontext beispielhafter Erläuterungen zur Kennzeichnung spezifischer Exemplare der mehrfach auftretenden Elemente genutzt.

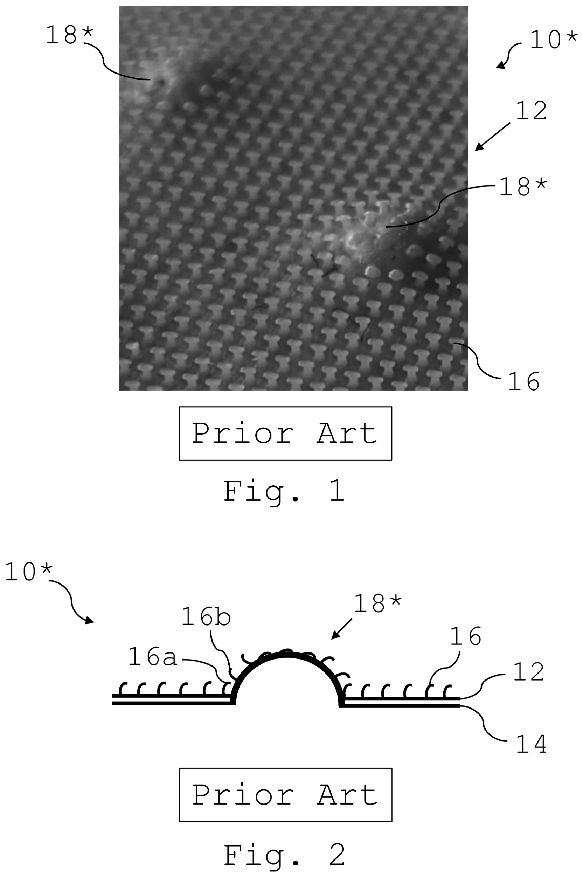

**Fig.** 1 zeigt ein bekanntes Verschlusssystem **10*** ausschnittsweise in einer perspektivischen Ansicht. Ein Trägerteil **12** weist in regelmäßigem Muster angeordnete Verschlusselemente **16** auf. Die Verschlusselemente 16 sind pilzförmig ausgebildet. Unterhalb des Trägerteils 12 ist ein Festlegeteil **14** angeordnet (in Figur 1 verdeckt; siehe **Fig. 2****).** Das Trägerteil 12 und das Festlegteil 14 sind über diskrete Schweißstellen **18*** miteinander verbunden. Beim Erzeugen der jeweiligen Schweißstellen 18* wurden jeweils einige der ursprünglich vorhandenen Verschlusselemente 16 zerstört (überschweißt). Zusätzlich ist durch die jeweilige Schweißstelle 18* eine Aufwölbung entstanden. Verschlusselemente 16 an der umlaufenden Flanke der jeweiligen Aufwölbung weisen dadurch insbesondere eine andere Orientierung auf als Verschlusselemente 16 in Bereichen abseits der Schweißstellen 18*. Die Haftfähigkeit des dargestellten bekannten Verschlusssystems 10* bzw. des dargestellten verschweißten Trägerteils 12 ist gegenüber einem entsprechenden, unverschweißt vorliegenden Trägerteil 12 nicht nur durch die tatsächlich überschweißten Verschlusselemente 16 vermindert, sondern auch aufgrund der im Vergleich zu den Dimensionen der Verschlusselemente 16 großen Aufwölbungen der Schweißstellen 18*. Auswirkungen dieser Aufwölbungen erstrecken sich in der Ebene des dargestellten bekannten Verschlusssystems 10* in alle Richtungen. Es ist insbesondere ersichtlich, dass durch die Schweißstellen 18* bei der Handhabung des Verschlusssystems 10* haptisch unangenehme Störstellen bedingt sind. Dies gilt umso mehr, da die vordringenden verschweißten Bereiche für gewöhnlich eine höhere Steifigkeit aufweisen als unverschweißte Bereiche des Trägerteils 12 und darüber hinaus möglicherweise scharfkantige Strukturen beim Verschweißen entstanden sind.

**Fig. 2** zeigt schematisch eine ausschnittsweise Seitenansicht eines weiteren bekannten Verschlusssystems 10*. Eine Schweißstelle 18* ist ähnlich der Schweißstelle 18* aus Fig. 1 ausgebildet. Über die Schweißstelle 18* werden ein Trägerteil 12 und ein Festlegeteil 14 miteinander verbunden. Auf dem Trägerteil 12 sind Verschlusselemente 16 angeordnet, welche vorliegend hakenförmig ausgebildet sind. Ursprünglich am Trägerteil 12 vorhandene Verschlusselemente 16 auf der Kuppe der durch die Schweißstelle 18* bedingten Aufwölbung wurden durch Überschweißen zerstört. Die Verschlusselemente 16 an den Flanken der Aufwölbung wurden beim Verschweißen zumindest aus ihrer ursprünglichen Orientierung ausgelenkt. Beispielhaft ist dargestellt, dass selbst das von der Verschweißung unmittelbar unbeeinflusste Verschlusselement **16a** an der Entfaltung seiner potentiellen Haftfähigkeit gehindert wird. Einerseits ist es von dem ausgelenkten Verschlusselement **16b** überdeckt, andererseits liegt es ggf. ohnehin in einer Blindzone, welche beim Herstellen einer Haftverbindung nur schwer erreicht werden kann. Die durch die Schweißstelle 18* erzeugte Aufwölbung erhebt sich deutlich über die freien Enden der Verschlusselemente 16 in den unverschweißten Bereichen.

Bekannt sind auch Verschlusssysteme 10*, bei welchen die durch die Schweißstellen 18* bedingten Aufwölbungen "nach unten" über das Festlegeteil 14 hinaus vordringen (nicht dargestellt). Dabei sind im Wesentlichen ebenfalls die bereits erläuterten Nachteile gegeben.

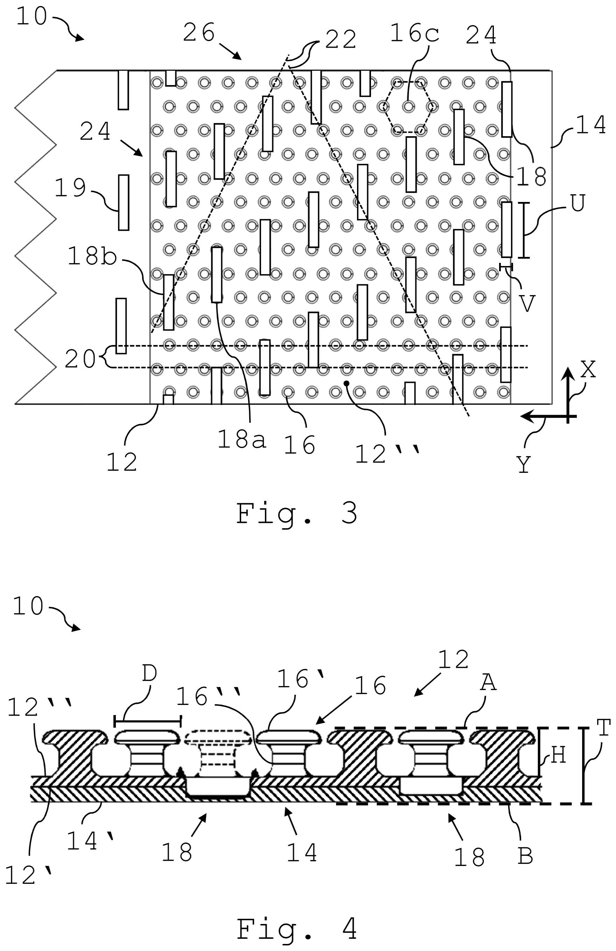

**Fig.** 3 zeigt ein erfindungsgemäßes Verschlusssystem 10 in einer Draufsicht. Verschlusselemente 16 sind dabei pilzförmig ausgebildet. Sie sind in einem regelmäßigen Muster auf einer Vorderseite **12"** eines Trägerteils 12 angeordnet. Insbesondere bilden die Verschlusselemente 16 dabei in einer Querrichtung Y des Trägerteils 12 Querreihen **20** aus. Zwei unmittelbar benachbarte Querreihen 20 weisen jeweils zueinander versetzte Verschlusselemente 16 auf. Dadurch ergibt sich eine Anordnung der Verschlusselemente 16 in sekundären Reihen **22.** Die sekundären Reihen 22 verlaufen schräg zu einer Längsrichtung X des Trägerteils 12. Betrachtet man eines der Verschlusselemente **16c,** welches vollständig von anderen Verschlusselementen 16 umgeben ist, wird durch die umgebenden Verschlusselemente 16 ein hexagonales Muster ausgebildet (über gestrichelte Linien angedeutet).

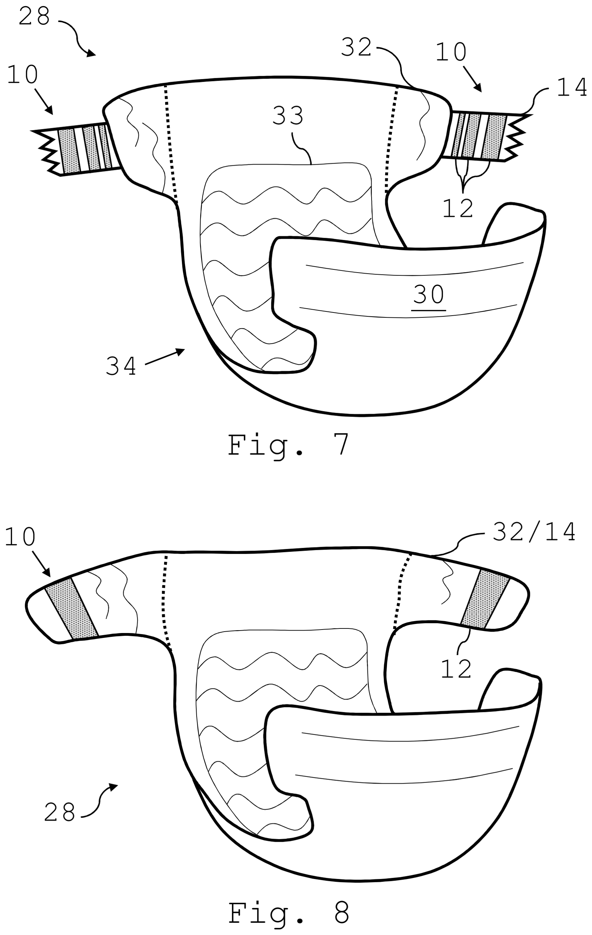

Schweißstellen 18 wurden während der Herstellung des in Fig. 3 dargestellten Verschlusssystems 10 allesamt gleichartig ausgebildet. Insbesondere gleichen sich deren jeweilige Ausdehnungen in der Längsrichtung X und der Querrichtung Y des Trägerteils 12. Durch Abtrennen von einem bandförmigen Verschlusssystem 10 (siehe **Fig. 8****)** wurden Schweißstellen 18 an Querrändern **26** des hier dargestellten Verschlusssystems 10 zerteilt. Im vorliegenden Zusammenhang werden dennoch alle am hier dargestellten Verschlusssystem 10 vorhandene Schweißstellen 18 weiterhin als gleichartig angesehen.

Die Schweißstellen 18 sind geradlinig ausgebildet und parallel zueinander angeordnet. Die Schweißstellen 18 (abseits der Querränder 26) weisen eine Längserstreckung **U** in Längsrichtung X des Trägerteils 12 auf. Im hier dargestellten Ausführungsbeispiel werden maximal zwei in der Längsrichtung X aufeinanderfolgende Verschlusselemente 16 von einer der Schweißstellen 18 erfasst.

Weiterhin weisen alle Schweißstellen 18 eine Breite **V** in der Querrichtung Y des Trägerteils 12 auf. Die Breite V entspricht hier in etwa einer maximalen Querausdehnung **D** (siehe **Fig. 4****)** eines der Verschlusselemente 16. So kann insbesondere die am Beispiel der Schweißstelle **18a** dargestellte Situation eingerichtet sein, wo nur ursprünglich vorhandene Verschlusselemente 16 durch die Schweißstelle 18a erfasst werden, welche auf einer geraden Linie (hier entlang der Längsrichtung X) aufeinanderfolgten. Weiterhin ist eine Positionierung der Schweißstellen 18 möglich, wie sie am Beispiel der Schweißstelle **18b** dargestellt ist, wo lediglich ein ursprünglich vorhandenes Verschlusselement 16 erfasst ist.

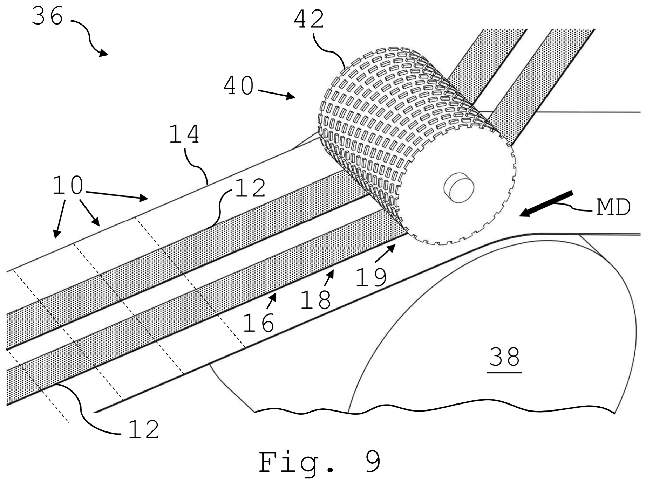

Die Schweißstellen 18 sind in einem regelmäßigen Muster an dem Verschlusssystem 10 angeordnet. Das vorliegende Schweißstellenmuster erstreckt sich dabei insbesondere über das Trägerteil 12 hinaus, wobei das Festlegeteil 14 Pseudoschweißstellen bzw. Einprägungen **19** aufweist. Die Einprägungen 19 können ggf. gezielt hervorgerufen worden sein, um beispielsweise das Erscheinungsbild und/oder die Steifigkeitsverteilung des Verschlusssystems 10 zu gestalten. Ebenfalls ist denkbar, dass die Einprägungen 19 aus der größeren Breite eines Ambosses **40** mit Schweißvorsprüngen **42** (siehe **Fig. 9****)** im Vergleich zum Trägerteil 12 resultieren.

Schweißstellen 18 an einem in der Darstellung von Fig. 3 rechtsseitigen Längsrand **24** überlappen diesen Längsrand 24 des Trägerteils 12 geringfügig. Dabei wird insbesondere ein Abstehen des Längsrandes 24 vom Festlegeteil 14 verhindert. Gleichermaßen gilt hier, dass die Fixierung des Längsrandes 24 ggf. gezielt erzeugt worden sein kann, oder sich beim dargestellten Verschlusssystem 10 zufällig ergeben haben kann.

Mehrere Schweißstellen 18 sind in Längsreihen (hier entlang der Längsrichtung X) angeordnet. Zwischen in Längsrichtung X aufeinanderfolgenden Schweißstellen 18 befindet sich jeweils zumindest ein Verschlusselement 16. Bei einer Betrachtung quer zu den Längsreihen (hier entlang der Querrichtung Y) können sich Schweißstellen 18 unterschiedlicher Längsreihen, insbesondere unmittelbar benachbarter Längsreihen, überlappen.

Beim in Fig. 3 dargestellten Verschlusssystem 10 wurden etwa 17 % der ursprünglich am Trägerteil 12 vorhandenen Verschlusselemente 16 überschweißt. In Ausführungsformen können insbesondere höchstens 15 % der ursprünglich vorhandenen Verschlusselemente 16 überschweißt worden sein (nicht im Detail dargestellt).

Die Darstellung der Verschlusselemente 16 und der Schweißstellen 18 ist in Fig. 3 zur besseren Veranschaulichung insbesondere überdimensioniert. Ein reales erfindungsgemäßes Verschlusssystem 10 kann insbesondere eine größere Anzahl von Verschlusselementen 16 aufweisen.

**Fig. 4** zeigt ausschnittsweise ein erfindungsgemäßes Verschlusssystem 10 in einer geschnittenen Seitenansicht. Ein Trägerteil 12 ist mit seiner Rückseite **12'** an einem Festlegeteil 14 angeordnet. Verschlusselemente 16 sind beispielhaft wie bei dem Verschlusssystem 10 aus Fig. 3 pilzförmig ausgebildet und in einem entsprechenden Muster angeordnet. Die Verschlusselemente 16 weisen jeweils eine Höhe **H** auf, welche vom jeweiligen freien Ende eines Verschlusselements 16 senkrecht auf die Vorderseite 12" des Trägerteils 12 gemessen wird. In der Schnittebene sind Verschlusselemente 16 einer ersten Querreihe 20 (siehe Fig. 3) angeordnet. In versetzter Anordnung sind dazwischen Verschlusselemente 16 einer zweiten Querreihe 20 dargestellt. Andeutungsweise ist ein Verschlusselement 16 einer dritten Querreihe 20 sichtbar.

Die Verschlusselemente 16 können insbesondere in je einen Kopf **16'** und je einen Stiel **16"** unterteilt werden. Die maximale Querausdehnung D befindet sich typischerweise am Kopf 16' eines jeweiligen Verschlusselementes 16, im vorliegenden Ausführungsbeispiel knapp unterhalb des freien Endes des jeweiligen Verschlusselementes 16. Im Falle von Verschlusselementen 16, welche an ihrem Stiel 16" eine größere Ausdehnung als am Kopf 16' aufweisen (nicht dargestellt, der Stiel verjüngt sich dann zum Kopf hin, sodass der Kopf dennoch übersteht) ist im vorliegenden Zusammenhang weiterhin die maximale Querausdehnung D im Bereich des Kopfes 16' maßgeblich. Beispielsweise könnten Verschlusselemente 16 an ihrem unteren, angebundenen, Ende einen sich stark verbreiternden Stiel 16" aufweisen. Manche dieser verbreiterten Stiele 16" könnten von einigen der Schweißstellen 18 teilweise überdeckt werden, ohne die Funktion der entsprechenden Verschlusselemente 16 zu beeinträchtigen (nicht im Detail dargestellt). In diesem Fall gelten die entsprechenden Verschlusselemente 16 weiterhin als nicht von Schweißstellen 18 erfasst.

In der Darstellung in Fig. 4 sind zwei Schweißstellen 18 sichtbar. Die Schweißstelle 18 auf der linken Seite erfasst ein ursprünglich in der dargestellten Schnittebene vorhandenes Verschlusselement 16. Dadurch ist, insbesondere lokal begrenzt, mehr Material an der linken Schweißstelle 18 vorhanden. Daraus ergibt sich insbesondere, dass die entsprechende Schweißstelle 18 eine Materialaufhäufung an der Vorderseite 12" des Trägerteils 12 und/oder, zumindest lokal, einen höheren Integrationsgrad zwischen dem Material des Trägerteils 12 und dem Material des Festlegeteils 14 aufweisen kann als die rechte Schweißstelle 18, welche im Bereich der Schnittebene in einem Zwischenraum zwischen Verschlusselementen 16 verläuft.

Der Großteil der Schweißstellen 18 erfasst im Verlauf ihrer jeweiligen Längserstreckung U (vgl. Fig. 3) typischerweise zumindest je ein Verschlusselement 16. Durch eine jeweilig vorhandene Materialmenge bedingte Unterschiede zwischen den Schweißstellen 18 sind in Bezug auf die Beurteilung der "Gleichartigkeit" verschiedener Schweißstellen 18 vernachlässigbar, da hierbei insbesondere auf die jeweiligen nominellen geometrischen Ausdehnungen abgestellt wird. Die in Fig. 4 dargestellten Schweißstellen 18 werden hier demnach insbesondere als gleichartig angesehen.

Über die freien Enden der Verschlusselemente 16 ist eine Referenzfläche **A** definiert. Im dargestellten Ausführungsbeispiel haben alle Verschlusselemente 16 dieselbe Höhe H. Sollten Verschlusselemente 16 unterschiedlicher Höhen H vorhanden sein, wird die Referenzfläche A anhand der höchsten Verschlusselemente bestimmt.

Über eine Rückseite **14'** des Festlegeteils 14 ist eine weitere Referenzfläche **B** definiert. Da die Schweißstellen 18 im vorliegend dargestellten Ausführungsbeispiel keine Aufwölbungen über die Rückseite 14' hinaus hervorrufen, entspricht die gesamte Rückseite 14' der Referenzfläche B.

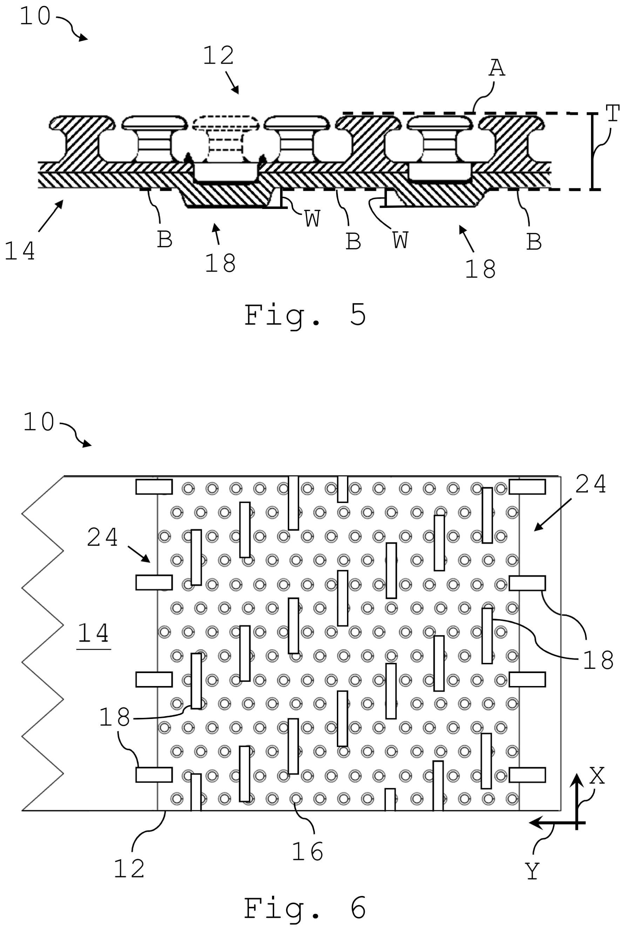

In Fällen in denen Schweißstellen 18 gewisse Aufwölbungen hervorrufen, werden zur Bestimmung der Referenzfläche B nur Bereiche neben den Schweißstellen 18, d. h. die unverschweißten Bereiche abseits der Aufwölbungen herangezogen (vgl. **Fig. 5****).**

Die Referenzflächen A, B sind zwei zueinander parallele Ebenen, zwischen welchen eine Dicke **T** gemessen wird. Erfindungsgemäß beträgt ein Überstand **W** (siehe Fig. 5) einer der jeweiligen Schweißstellen 18 bzw. der durch die jeweilige Schweißstelle 18 hervorgerufenen Aufwölbung über die jeweilige nächstgelegene Referenzfläche A, B hinaus höchstens 20 % der Dicke T. In Fig. 5 ist der besseren Erkennbarkeit halber der Überstand W nicht maßstäblich, sondern vergrößert, dargestellt.

Der Überstand W wird dabei stets von dem Punkt der Schweißstelle 18 gemessen, der an dieser Schweißstelle 18 am weitesten aus dem zwischen den Referenzflächen A, B gelegenen Bereich hinausragt, hier über die Referenzfläche B. Von diesem Punkt wird senkrecht auf die nächstgelegene Referenzfläche A, B gemessen.

Besonders bevorzugt dringen Schweißstellen 18 nicht aus dem Bereich zwischen den Referenzflächen A, B über eine der Referenzflächen A, B hinaus. Dies ist in Fig. 4 dargestellt. Dadurch ist ein haptisch besonders angenehmes (glattes) Verschlusssystem 10 gegeben, wobei kein aufwölbungsbedingter Haftfähigkeitsverlust verglichen mit einem unverschweißt vorliegenden Trägerteil 12 auftritt. Bei einem direkten Vergleich von Fig. 4 mit den Fign. 1 und 2 ist dies deutlich ersichtlich.

**Fig. 5** zeigt ein weiteres erfindungsgemäßes Verschlusssystem 10, wobei Schweißstellen 18 einen jeweiligen Überstand W über eine Referenzfläche B hinaus aufweisen. Anderweitig entspricht das Verschlusssystem 10 in Fig. 5 im Wesentlichen dem Verschlusssystem 10 in Fig. 4. Ersichtlich ist, dass insbesondere zur Bestimmung der Referenzebene B lediglich Bereiche neben den Schweißstellen 18, abseits der durch die Schweißstellen 18 hervorgerufenen Aufwölbungen, herangezogen werden. Gleichermaßen werden überschweißte Verschlusselemente 16 bei der Bestimmung der Referenzfläche A nicht berücksichtigt. Der jeweilige Überstand W bezeichnet den Abstand des am weitesten vordringenden Bereichs der jeweiligen Schweißstelle 18 (bzw. der durch die Schweißstelle 18 hervorgerufenen Aufwölbung) zu der nächstgelegenen Referenzfläche B. Der Überstand W beträgt höchstens 20 % der Dicke T. Bevorzugt beträgt der Überstand W deutlich weniger als 20 % der Dicke T, insbesondere höchstens 5 % der Dicke T. In der ohnehin nicht als maßstäblich zu betrachtenden Fig. 5 ist ein jeweiliger Überstand W verglichen mit der Dicke T zur Veranschaulichung insbesondere überdimensioniert dargestellt.

**Fig. 6** zeigt ein weiteres erfindungsgemäßes Verschlusssystem 10 mit einem Trägerteil 12 und einem Festlegeteil 14. Im Unterschied zu dem Verschlusssystem 10 aus Fig. 3, wurden hier Schweißstellen 18 zur Fixierung der Längsränder 24 des Trägerteils 12 bereits bei der Auslegung des Verschlusssystems 10 vorgesehen. Ein entsprechendes Herstellungsverfahren erfordert dabei ggf. ein höheres Maß an Präzision bzw. ist weniger flexibel in der Verwendung unterschiedlich breiter Trägerteile 12 mit demselben Amboss 40 einer Ultraschallschweißanlage **36** (siehe Fig. 9).

Schweißstellen 18 an den Längsrändern 24 des Trägerteils 12 überlappen diese zum Festlegeteil 14 hin. Die Schweißstellen 18 an den Längsrändern 24 weisen vorzugsweise eine andere geometrische Form auf als innenliegende Schweißstellen 18. Insbesondere können sie eine größere Ausdehnung in der Querrichtung Y als in der Längsrichtung X des Trägerteils 12 aufweisen.

Wie aus Fig. 6 ersichtlich ist, sind Schweißstellen 18 am rechten Längsrand 24 des Trägerteils 12 derart angeordnet, dass keines der dortigen Verschlusselemente 16 erfasst ist. Eine derartige Anordnung der Schweißstellen 18 kann im Rahmen der Auslegung des Verschlusssystems 10 vorgesehen werden. Am linken Längsrand 24 erfasst demgegenüber eine jeweilige Schweißstelle 18 jeweils ein ursprünglich vorhandenes Verschlusselement 16. Um alle Verschlusselemente 16 an den Längsrändern 24 des Trägerteils 12 erhalten zu können, könnten die Schweißstellen 18 des linken Längsrandes 24 insbesondere in der Längsrichtung X gegenüber den Schweißstellen 18 am rechten Längsrand 24 versetzt angeordnet werden.

**Fig. 7** zeigt einen erfindungsgemäßen Hygieneartikel **28,** in Form einer Windel, mit einem saugfähigen Kern **33** und zwei erfindungsgemäßen Verschlusssystemen 10. Eine, insbesondere Vliesstoff aufweisende, Landing Zone der Windel dient als Gegenstück **30** für beide Verschlusssysteme 10.

Im dargestellten Ausführungsbeispiel ist ein jeweiliges Festlegeteil 14 der Verschlusssysteme 10 als Lasche ausgebildet, welche wiederum an einem Windelohr **32** angebunden ist. Die Anbindung des Festlegeteils 14 an das Windelohr 32 kann beispielsweise ebenfalls über eine Verschweißung realisiert sein. Das Windelohr 32 kann wiederum an einen Hauptkörper **34** der Windel angebunden sein, oder monolithisch mit diesem ausgebildet sein.

Ein jeweiliges Festlegeteil 14 trägt hier mehrere Trägerteile 12. Die Trägerteile 12 eines Festlegeteils 14 sind in Streifen an dem Festlegeteil 14 angeordnet und entlang der Querrichtung Y (siehe Fig. 3 oder 6) der Trägerteile 12 beabstandet. Verschlusselemente 16 und Schweißstellen 18 sind hier nicht im Detail dargestellt (siehe dazu Fign. 3 bis 6).

Die verschiedenen Trägerteile 12 können hinsichtlich ihrer Ausdehnungen in der Querrichtung Y und/oder der Art ihrer Verschlusselemente 16 insbesondere gleich oder verschieden ausgebildet sein. Typischerweise erstrecken sich die Trägerteile 12 in Längsrichtung X (siehe Fig. 3 oder 6) über das gesamte Festlegeteil 14.

Zwei Verschlusssysteme 10 eines Hygieneartikels 28 müssen nicht notwendigerweise gleich ausgebildet sein, obgleich dies bei Windeln typischerweise der Fall ist.

Mittels der dargestellten mehreren Trägerteile 12 pro Verschlusssystem 10 kann insbesondere erreicht werden, dass die das Festlegeteil 14 bildende Lasche eine angenehmere Haptik aufweist, insbesondere weicher und biegsamer ist, als das Festlegeteil 14 eines Verschlusssystems 10 mit einem in Querrichtung Y durchgehenden Trägerteil 12 gleicher Gesamtfläche. Gleichmaßen kann das Öffnungsverhalten durch die Anpassung des Verlaufs der beim Öffnen benötigten Kräfte angepasst werden. Derart kann der wahrgenommene Handhabungskomfort gesteigert werden.

Unter Umständen ist an den jeweiligen Längsrändern 24 (siehe Fig. 6) der jeweiligen Trägerelemente 12 darüber hinaus eine geringfügig höhere Anhaftung an dem Gegenstück 30 (hier Landing Zone der Windel) gegeben als im Restbereich eines jeweiligen Trägerteils 12. Dies kann beispielsweise daraus folgen, dass Verschlusselemente 16 am Längsrand 24 einseitig einen größeren Wirkspielraum haben (ggf. mehr Verhakungen mit Schlaufen eines Gegenstücks 30 erzeugen können). Die Haftfähigkeit eines Verschlusssystems 10 kann durch eine fragmentierte Trägerteilanordnung ggf. erhöht werden, ohne die Gesamtzahl der Verschlusselemente 16 zu erhöhen.

In Ausführungsformen kann ein Windelohr 32 selbst ein Festlegeteil 14 darstellen. Dies ist in **Fig. 8** an einem weiteren Ausführungsbeispiel eines Hygieneartikels 28 dargestellt. Dabei sind Trägerteile 12 mittels Schweißstellen 18 (siehe Fign. 3 bis 6) direkt auf dem Windelohr 32 angebracht.

**Fig. 9** zeigt schematisch eine perspektivische, ausschnittsweise Ansicht einer Ultraschallschweißanlage 36, welche insbesondere zwei Schweißwerkzeuge aufweist. Eines der Schweißwerkzeuge ist eine Sonotrode **38,** während das andere Schweißwerkzeug einen Amboss 40 darstellt. Sonotrode 38 und Amboss 40 wirken zur Herstellung einer jeweiligen Schweißstelle 18 (siehe auch Fign. 3 bis 6) zusammen. Die Sonotrode 38 ist hier walzenförmig und mit einer glatten Umfangsfläche ausgebildet. Der Amboss 40 ist vorzugsweise angetrieben oder frei um seine Zylinderachse drehbar an der Ultraschallschweißanlage 36 gelagert. Die Sonotrode 38 kann ebenfalls drehbar ausgebildet sein. Alternativ kann die Sonotrode 38 insbesondere eine flache Wirkoberfläche aufweisen, an welcher vorzugsweise die Rückseite 14' (siehe Fig. 4) des Festlegeteils 14 entlanggleiten kann (nicht näher dargestellt).

Fig. 9 zeigt ein bandförmiges Festlegeteil 14 sowie zwei bandförmige Trägerteile 12, welche entlang einer Maschinenrichtung **MD** zusammengeführt und miteinander verschweißt werden. Die Maschinenrichtung MD entspricht vorzugsweise der jeweiligen Längsrichtung X (siehe Fig. 3 oder 6) der Trägerteile 12. Die Trägerteile 12 können, müssen aber nicht notwendigerweise, gleichartig ausgebildet sein. Über gestrichelte Linien ist angedeutet, dass durch Zuschneiden kleinteiligere Verschlusssysteme 10 aus dem bandförmigen Verschlusssystem 10 erhalten werden können. Ein Zuschneiden muss nicht notwendigerweise wie angedeutet erfolgen. Insbesondere könnte eines der dargestellten Verschlusssysteme 10 parallel zur Maschinenrichtung MD geschnitten werden, wobei resultierende Verschlusssysteme 10 nur je ein Trägerteil 12 auf je einem Festlegeteil 14 aufweisen würden.

Der Amboss 40 weist diskrete Schweißvorsprünge 42 zur Erzeugung der Schweißstellen 18 auf. Die Schweißvorsprünge 42 sind im dargestellten Ausführungsbeispiel so angeordnet, dass ein Schweißmuster ähnlich dem in Fig. 3 dargestellten Schweißmuster erzeugt wird. Die Darstellung der Schweißvorsprünge 42 erfolgt in (der ohnehin nicht als maßstäblich zu betrachtenden) Fig. 9 zur Veranschaulichung insbesondere überdimensioniert. Die Schweißvorsprünge 42 sind hier insbesondere regelmäßig am Amboss 40 angeordnet. Auch weisen die Schweißvorsprünge 42 hier jeweils identische Ausdehnungen, insbesondere auch identische Vorsprunghöhen, auf, um die Ausbildung gleichartiger Schweißstellen 18 zu ermöglichen. Schweißstellen 18, Einprägungen 19 und Verschlusselemente 16 sind in Fig. 9 nicht im Detail dargestellt.

Der Amboss 40 kann als einstückiges Bauteil vorliegen oder mehrere Komponenten aufweisen. Insbesondere kann der Amboss 40 modular erweiterbar oder konfigurierbar sein, beispielsweise um unterschiedlich viele Bahnen von Trägerteilen 12 gleichzeitig zu bearbeiten und/oder Schweißmuster anzupassen.

Mittels des in Fig. 9 dargestellten Prinzips lässt sich ein erfindungsgemäßes Verfahren zum Herstellen eines Verschlusssystems 10 in rationeller Weise durchführen. Insbesondere lassen sich dabei hohe Prozessgeschwindigkeiten erreichen. Beispielsweise kann eine Vorschubgeschwindigkeit von Trägerteilen 12 und Festlegeteil 14 in der Maschinenrichtung MD mehr als 250 m/min betragen.

Unter Vornahme einer Zusammenschau der Figuren der Zeichnung betrifft die Erfindung ein Verschlusssystem 10, aufweisend ein Trägerteil 12 mit einer Rückseite 12' und einer Vorderseite 12". Von der Vorderseite 12" stehen in einem regelmäßigem Muster Verschlusselemente 16, 16a, 16b, 16c, insbesondere mit einer Höhe H von höchstens 1 mm, bevorzugt höchstens 0,5 mm, vor. Ein Festlegeteil 14 ist an der Rückseite 12' des Trägerteils 12 angeordnet. Das Trägerteil 12 und das Festlegeteil 14 sind durch mehrere Schweißstellen 18, 18a, 18b bereichsweise miteinander verbunden.

Neben einer jeweiligen Schweißstelle 18, 18a, 18b (d. h. außerhalb des von der jeweiligen Schweißstelle 18, 18a, 18b erfassten und ggf. deformierten Bereichs) definieren die freien Enden der Verschlusselemente 16, 16a, 16b, 16c und eine vom Trägerteil 12 abgewandte Rückseite 14' des Festlegeteils 14 jeweils eine Referenzfläche A, B. Ein jeweiliger Überstand W der Schweißstellen 18, 18a, 18b, welcher aus einem Bereich zwischen den Referenzflächen A, B hinausragt, beträgt höchstens 20 %, bevorzugt höchstens 10 %, besonders bevorzugt höchstens 5 %, einer zwischen den Referenzflächen A, B gemessenen Dicke T des Verschlusssystems 10 zu der jeweilig dem Überstand W nächstliegenden Referenzfläche A, B. Insbesondere stehen zumindest einige, vorzugsweise alle, der Schweißstellen 18, 18a, 18b nicht aus dem Bereich zwischen den Referenzflächen A, B vor.

Wenigstens eine der Schweißstellen 18, 18a, 18b, die von allen Rändern 24, 26 des Trägerteils 12 beabstandet ist, erfasst wenigstens ein ursprünglich am Trägerteil 12 vorhandenes Verschlusselement 16, 16a, 16b, 16c und höchstens 10, insbesondere höchstens 5, vorzugsweise höchstens 3, ursprünglich am Trägerteil 12 vorhandene Verschlusselemente 16, 16a, 16b, 16c.

Die Erfindung betrifft auch ein Verfahren zum Herstellen eines erfindungsgemäßen Verschlusssystems 10 und einen Hygieneartikel 40, insbesondere in Form einer Windel, der wenigstens ein erfindungsgemäßes Verschlusssystem 10 aufweist.

### Bezugszeichenliste

| | | | |
|---|---|---|---|
| 10* | bekanntes Verschlusssystem | 32 | Windelohr |
| 10 | Verschlusssystem | 33 | Kern |
| 12 | Trägerteil | 34 | Hauptkörper |
| 12' | Rückseite Trägerteil | 36 | Ultraschallschweißanlage |
| 12" | Vorderseite Trägerteil | 38 | Sonotrode |
| 14 | Festlegeteil | 40 | Amboss |
| 14' | Rückseite Festlegeteil | 42 | Schweißvorsprung |
| 16 | Verschlusselement | | |
| 16a | Verschlusselement | X | Längsrichtung |
| 16b | Verschlusselement | Y | Querrichtung |
| 16c | Verschlusselement | | |
| 16' | Kopf | U | Längserstreckung |
| 16" | Stiel | V | Breite |
| 18* | bekannte Schweißstelle | W | Überstand |
| 18 | Schweißstelle | | |
| 18a | Schweißstelle | D | Querausdehnung |
| 18b | Schweißstelle | T | Dicke |
| 19 | Einprägung | H | Höhe |
| 20 | Querreihe | | |
| 22 | sekundäre Reihe | A | Referenzebene |
| 24 | Längsrand | B | Referenzebene |
| 26 | Querrand | | |
| 28 | Hygieneartikel | MD | Maschinenrichtung |
| 30 | Gegenstück | | |

## Patentansprüche

1. Verschlusssystem (10), aufweisend ein Trägerteil (12) mit einer Rückseite (12') und einer Vorderseite (12"), von der in einem regelmäßigem Muster Verschlusselemente (16, 16a, 16b, 16c), insbesondere mit einer Höhe (H) von höchstens 1 mm, bevorzugt höchstens 0,5 mm, vorstehen, und ein Festlegeteil (14), das an der Rückseite (12') des Trägerteils (12) angeordnet ist,
wobei das Trägerteil (12) und das Festlegeteil (14) durch mehrere Schweißstellen (18, 18a, 18b) bereichsweise miteinander verbunden sind, wobei neben einer jeweiligen Schweißstelle (18, 18a, 18b) die freien Enden der Verschlusselemente (16, 16a, 16b, 16c) und eine vom Trägerteil (12) abgewandte Rückseite (14') des Festlegeteils (14) jeweils eine Referenzfläche (A, B) definieren,
wobei ein Überstand (W) der Schweißstellen (18, 18a, 18b) über die Referenzflächen (A, B) höchstens 20 %, bevorzugt höchstens 10 %, besonders bevorzugt höchstens 5 %, einer zwischen den Referenzflächen (A, B) gemessenen Dicke (T) des Verschlusssystems (10) beträgt, insbesondere wobei die Schweißstellen (18, 18a, 18b) nicht über die Referenzflächen (A, B) überstehen,
und wobei wenigstens eine der Schweißstellen (18, 18a, 18b), die von Rändern (24, 26) des Trägerteils (12) beabstandet ist, wenigstens ein ursprünglich am Trägerteil (12) vorhandenes Verschlusselement (16, 16a, 16b, 16c) und höchstens 10, insbesondere höchstens 5, vorzugsweise höchstens 3, ursprünglich am Trägerteil (12) vorhandene Verschlusselemente (16, 16a, 16b, 16c) erfasst.

2. Verschlusssystem (10) nach Anspruch 1, wobei die Schweißstellen (18, 18a, 18b) allesamt gleichartig ausgebildet und/oder in einem regelmäßigen Muster angeordnet sind.

3. Verschlusssystem (10) nach einem der vorhergehenden Ansprüche, wobei zumindest einige der Schweißstellen (18, 18a, 18b), insbesondere diejenigen, welche ursprünglich vorhandene Verschlusselemente (16, 16a, 16b, 16c) erfassen, insbesondere alle Schweißstellen (18, 18a, 18b) sich geradlinig erstrecken, vorzugsweise wobei die geradlinigen Schweißstellen (18, 18a, 18b) parallel zueinander ausgerichtet sind.

4. Verschlusssystem (10) nach einem der vorhergehenden Ansprüche, wobei die Verschlusselemente (16, 16a, 16b, 16c) in einem hexagonalen Muster und/oder in schräg zu einer Längsrichtung (X), insbesondere schräg zu geradlinigen Schweißstellen (18, 18a, 18b), verlaufenden Reihen angeordnet sind.

5. Verschlusssystem (10) nach einem der vorhergehenden Ansprüche, wobei höchstens 20 %, insbesondere höchstens 15 %, der ursprünglich vorhandenen Verschlusselemente (16, 16a, 16b, 16c) von Schweißstellen (18, 18a, 18b) erfasst sind.

6. Verschlusssystem (10) nach einem der vorhergehenden Ansprüche, wobei eine Breite (V) der Schweißstellen (18, 18a, 18b) höchstens dem Abstand zweier benachbarter Verschlusselemente (16, 16a, 16b, 16c) und/oder höchstens einer Querausdehnung (D) eines Verschlusselements (16, 16a, 16b, 16c), insbesondere eines überschweißten Verschlusselements (16, 16a, 16b, 16c), entspricht.

7. Verschlusssystem (10) nach einem der vorhergehenden Ansprüche, wobei zumindest einige, insbesondere alle, Schweißstellen (18, 18a, 18b) in einer Längsrichtung (X) eine Längserstreckung (U) aufweisen, die wenigstens dem Abstand zweier in der Längsrichtung (X) unmittelbar aufeinanderfolgender Verschlusselemente (16, 16a, 16b, 16c) und/oder höchstens dem Abstand dreier in der Längsrichtung (X) unmittelbar aufeinanderfolgender Verschlusselemente (16, 16a, 16b, 16c) entspricht.

8. Verschlusssystem (10) nach einem der vorhergehenden Ansprüche, wobei die von einer jeweiligen Schweißstelle (18, 18a, 18b) erfassten ursprünglich vorhandenen Verschlusselemente (16, 16a, 16b, 16c) entlang einer geraden Linie aufeinanderfolgen.

9. Verschlusssystem (10) nach einem der vorhergehenden Ansprüche, wobei die Schweißstellen (18, 18a, 18b) in Längsreihen angeordnet sind, und wobei - bei Betrachtung quer zu den Längsreihen - Schweißstellen (18, 18a, 18b) unmittelbar benachbarter Längsreihen einander in der Längsrichtung (X) überlappen.

10. Verschlusssystem (10) nach einem der vorhergehenden Ansprüche, weiterhin aufweisend ein Gegenstück (30) zum Zusammenwirken mit den Verschlusselementen (16, 16a, 16b, 16c), wobei durch das Überschweißen der Verschlusselemente (16, 16a, 16b, 16c) eine Festigkeit der Verbindung des Trägerteils (12) mit dem Gegenstück (30) um höchstens 20 % verringert ist gegenüber einem gleichartigen Trägerteil (12), bei dem alle Verschlusselemente (16, 16a, 16b, 16c) intakt sind.

11. Verschlusssystem (10) nach einem der vorhergehenden Ansprüche, wobei die Verschlusselemente (16, 16a, 16b, 16c) an den freien Enden Köpfe (16') aufweisen, die zumindest in einer Richtung, insbesondere allseitig, über einen Stiel (16") des jeweiligen Verschlusselements (16, 16a, 16b, 16c) vorstehen.

12. Verschlusssystem (10) nach einem der vorhergehenden Ansprüche, weiterhin aufweisend wenigstens ein weiteres Trägerteil (12), das wie das erste Trägerteil (12) mit dem Festlegteil (14) verbunden ist, insbesondere wobei sich die mehreren Trägerteile (12) parallel zueinander erstrecken.

13. Hygieneartikel (28), insbesondere Windel, mit einem Verschlusssystem (10) nach einem der vorhergehenden Ansprüche, insbesondere einem Verschlusssystem (10) nach Anspruch 12.

14. Verfahren zum Herstellen eines Verschlusssystems (10) nach einem der Ansprüche 1 bis 12, wobei zum Verschweißen ein Trägerteil (12) und ein Festlegteil (14) zwischen einem, vorzugsweise rotierenden, Amboss (40) und einer Sonotrode (38) einer Ultraschallschweißanlage (36) durchgeführt werden.

15. Verfahren nach Anspruch 14, wobei beim Verschweißen der Amboss (40) an dem Trägerteil (12) anliegt und die Sonotrode (38) an dem Festlegeteil (14) anliegt.

16. Verfahren nach Anspruch 14 oder 15, wobei der Amboss (40) mehrere, insbesondere in Umfangsrichtung voneinander beabstandete, Schweißvorsprünge (42) aufweist.
